# EUROPEAN PATENT SPECIFICATION

(11) **EP 0 640 575 B1**
(45) Date of publication and mention of the grant of the patent: **14.05.1997**
(21) Application number: 94202421.7
(22) Date of filing: 24.08.1994
(51) Int. Cl.: C07C 2/58, B01J 29/90

(54) **Process for upgrading a paraffinic feedstock**
Verfahren zur Aufwertung einer paraffinischen Beschickung
Procédé pour la valorisation d'une charge paraffinique

(30) Priority: 26.08.1993 EP 93202515
(43) Date of publication of application: 01.03.1995
(73) Proprietor: SHELL INTERNATIONALE RESEARCH MAATSCHAPPIJ B.V., 2596 HR Den Haag (NL)
(72) Inventor: Van Brugge, Paulus Theodorus Maria, NL-1031 CM Amsterdam (NL); De Groot, Christoffel, NL-1031 CM Amsterdam (NL); Mesters, Carolus Matthias Anna Maria, NL-1031 CM Amsterdam (NL); Peferoen, Danny Gaston Rene, NL-1031 CM Amsterdam (NL)

(56) References cited:
- FR-A- 2 631 956
- US-A- 3 851 004
- US-A- 4 508 836
- "Longman Dictionary of the English Language"p.363;"Zeolite Molecular Sieves,Structure,Chemistry and Use"by Donald W, Breck,p.348-351, 1984.

## Description

The present invention relates to a process for upgrading a paraffinic feedstock. More specifically the invention relates to a process for alkylation of a paraffinic feedstock by the condensation of paraffins with olefins.

The production of highly branched hydrocarbons such as trimethylpentane is important by virtue of their use as gasoline blending components of high octane number. Traditional production of highly branched hydrocarbons is by condensation of isobutane with light olefins, usually butenes but sometimes mixtures of propene, butenes and possibly pentenes using large quantities of conventional strong liquid acid catalysts, such as hydrofluoric or sulphuric acid. An emulsion of immiscible acid and hydrocarbon is agitated to emulsify the catalyst and reactant and refrigerated to control the highly exothermic reaction. By fine control of a complex interrelation of process variables high quality alkylate production may be maintained. The acid is recycled after use. It is desirable to use a process which is less hazardous and toxic and environmentally more acceptable.

Processes have been proposed to overcome these problems by using solid acids as catalysts. For example, FR-A-2 631 956 describes a process for the catalytic alkylation of isobutane or isopentane with a C₃-C₄ olefin in the presence of a zeolite beta solid acid catalyst.

However, paraffin olefin condensation yields both desired alkylate and undesired oligomerisation product. When catalysing alkylation with solid acids it has been found that hydrocarbonaceous deposit formation causes progressive deactivation of the catalyst. Regeneration techniques are however known for removal of hydrocarbonaceous deposits from solid catalysts to restore catalyst activity typically by raising the catalyst to elevated temperatures and oxidising the deposits but, in US-A-3 851 004, certain catalyst compositions comprising at least one hydrogenation agent of nickel, ruthenium, rhodium, palladium and platinum and a three-dimensional crystalline zeolitic molecular sieve having a pore diameter large enough to adsorb ortho-diethylbenzene, an alkali metal content of less than 3.5 %w on a solids basis and a silica to alumina molar ratio of at least 2, are regenerated by contacting the catalyst compositions, preferably in the absence of feed olefin, at a temperature of from 80 °F to 572 °F (26.6 °C to 300 °C) with a liquid solution of hydrogen in a saturated hydrocarbon having from 4 to 12 carbon atoms, the solution containing at least 0.1 mol% dissolved hydrogen.

Nevertheless rapid deactivation of the solid acid catalysts compared with liquid acid catalysts is a disadvantage.

There is a need for an environmentally acceptable paraffin/olefin alkylation process which selectively yields highly branched alkylate at an acceptable rate for prolonged periods on stream, and which allows flexibility in term of the olefin employed.

Copending European patent application numbers 92201015 and 92201016 disclose processes for upgrading a paraffinic feedstock comprising supplying feedstock and olefin at specified paraffin to olefin ratio to a reactor containing a solid acid catalyst, preferably a zeolite beta catalyst and subsequently removing the upgraded product wherein the processes are operated at high olefin conversion in respectively internal and external circulation reactors having respectively a high variance in residence time distribution of liquid reactor contents and a high extent of external circulation of liquid reactor contents. It has been found that under these conditions alkylation products are obtained for a catalyst lifetime considerably greater than that using known solid acid catalysed processes although at a lower rate of production of alkylate in comparison with techniques using liquid acids.

Whilst the use of regeneration techniques to restore catalyst activity in a cyclic alkylation process may increase the rate of alkylate formation averaged over the reaction-regeneration cycle, standard techniques for regeneration of hydrocarbon conversion catalysts such as burning of hydrocarbonaceous deposits in air would cause irreversible dealumination of certain zeolitic catalysts such as the preferred zeolite beta catalyst, making an alkylation process using zeolite beta containing catalyst an economically unattractive option. In J. Catalysis 124 (1990) 217-223 it is shown that the thermal stability of zeolite beta during exposure of a sample thereof to air at elevated temperature is poor: during a five hour period at 500 °C the silicon-to-aluminium atomic ratio in the framework increases significantly accompanied by an increase in the amount of octahedral-coordinated alumina, which effects result in an irretrievable loss of the alkylation activity of the zeolite. This indicates that elevated temperature techniques for regeneration of zeolite beta catalysts are of limited benefit.

It has now surprisingly been found that condensation of paraffins with olefins may be achieved using a solid acid catalysed alkylation process achieving an acceptable rate of production of product alkylate at high selectivity by using a catalyst comprising zeolite beta crystals wherein removal of hydrocarbonaceous deposits is achieved in a manner effective at non-extreme temperatures. Moreover it has surprisingly been found that by use of this regeneration technique, zeolite beta catalyst may be regenerated to substantially full activity even at extreme temperatures apparently without occurrence of framework dealumination effects.

Accordingly the present invention provides a cyclic process for catalytically alkylating a paraffinic feedstock and for upgrading the activity of aged catalyst comprising the steps a) of supplying feedstock and olefin at a paraffin to olefin ratio greater than 5 v/v to a reactor containing a solid acid catalyst and removing effluent comprising alkylate product and b) of exposing the catalyst to a hydrogenating medium and removing a hydrocarbonaceous effluent wherein the catalyst comprises zeolite beta crystals.

Reference herein to hydrocarbonaceous deposits is to compounds or radicals formed during the catalysed condensation of paraffin with olefin(s), which compounds or radicals have a semi permanent deactivating effect on the catalyst. It is speculated that these deposits comprise oligomerisation products and fragments thereof formed and retained within the zeolite pores. In the absence of an understanding of the nature of the deposits and their manner of association it is not possible to systematically approach the problem of inhibiting their formation or their deactivating effect, or of effecting their removal in a manner non-disruptive to the condensation reaction.

Reference herein to alkylate is to a mixed condensation product of paraffin with olefin(s), and by the term oligomerisation product is meant a condensation product of a plurality of olefin molecules. Alkylate is characterised by a higher motor octane number (MON) than oligomerisation product. Typically alkylate comprising highly branched paraffins with 5 to 12 carbon atoms has a MON of 86 or more, for example in the range 90 to 94 whilst a corresponding oligomerisation product may comprise a mixture of paraffinic or olefinic hydrocarbons typically of MON of less than 85, for example in the range 80 to 82. Moreover, both high and low quality alkylate are characterized by a lower sensitivity, i.e. difference in MON and RON values, than that of oligomerisate.

Reference herein to hydrogenation medium is to a medium which achieves transfer of hydrogen to the catalytic environment such that a hydrocarbonaceous effluent is obtained derived from the hydrocarbonaceous deposit. Without wishing to be limited to a particular theory it would appear that a medium which does not effectively achieve transfer of hydrogen to the catalytic environment would result in insufficient or substantially no effluent being obtained derived from the hydrocarbonaceous deposit. Insufficient effluent may be obtained as gaseous thermally decomposed deposits, of which more the carbon-rich counterpart is not hydrogenated, but retained on the catalyst. It would also appear that effective transfer takes place under conditions such that thermal decomposition of hydrocarbonaceous deposits is accompanied by hydrogenation of decomposed deposits at a rate in excess of rate of polymerisation thereof. The rate of hydrogenation in step b) would therefore be appropriately controlled by means of prevailing conditions and concentration of hydrogen available in the hydrogenating medium to take part in hydrogenation, for a given rate of thermal degradation of deposits in step b). The efficiency of the hydrogenation step may be determined from the composition of the hydrocarbonaceous effluent of step b) which preferably comprises lower saturated hydrocarbons derived from hydrogenated fragments of hydrocarbonaceous deposits. The hydrogenating medium suitably comprises a hydrogenating fluid which may be gaseous or liquid and suitably operates together with a hydrogenating function such as a hydrogenating metal component for example a Group VIII metal function which may be chemically associated with the solid acid alkylation catalyst, for example by ion exchange, admixed with the catalyst as separate particles on a suitable carrier or which may be brought into physical association with the catalyst for the duration of the deposits removal step. Preferably a hydrogenating metal function selected from platinum, palladium and nickel is incorporated in the catalyst.

Preferably the hydrogenating fluid comprises hydrogen, most preferably in gaseous form, optionally in admixture with an inert gaseous or liquid diluent or reactive hydrocarbon, for example with a gaseous lower alkane such as butane or with a reactive aromatic such as toluene, benzene or light reformate, in a ratio of 99:1 to 1:99 v/v. A suitable cheap source of hydrogenation gas may be a refinery dry gas typically comprising hydrogen, methane, ethane, ethene and a trace amount of propene, which may be essentially free of hydrogenating metal poisons such as hydrogen sulphide. It is in some instances desirable to moderate the activity of a hydrogenating metal function, whereby a trace amount of sulphur may be incorporated either on the catalyst or, in the form of hydrogen sulphide for example, as part of the hydrogenating fluid.

The process may be carried out in any suitable reactor or reactor configuration. Operation in fixed bed or slurry phase is preferred. The process may be operated in batchwise or continuous manner. Multiple reactors may be operated in series with respect to paraffinic feed and effluent lines, using split feed of olefin to each individual reactor, for improved alkylate quality. Preferably the process is operated in continuous manner advantageously enabling continuous production of alkylate. For example multiple fixed or slurry phase reactors may be operated with parallel feed and effluent lines and out of phase with respect to the cyclic process such as in a swing bed system whereby step a) of the process is in operation in at least one of the catalyst beds at any given time. Alternatively a slurry phase reactor may be operated continually in step a) of the process for example by use of a continuous regeneration system whereby a proportion of catalyst is continuously or periodically withdrawn from the slurry reactor to a separate vessel for operation of step b) and returned to the slurry reactor for operation of step a).

A portion of effluent comprising unreacted feedstock and alkylate from step a) may be retained in the system and recycled. Preferably effluents from steps a) and b) are combined and distilled whereby substantially only unreacted feed recycled, and may be the concentration of alkylate in the recycle stream is controlled. Alternatively effluent from step b) may be removed from the system via a separate effluent line. Where the hydrogenated deposit and/or the hydrogen rich light gas fraction finds application as feed for chemical processes or as a chemical product the latter case is preferred.

Introduction of hydrogenating fluid may be continuous or batchwise with continuous or batchwise withdrawal of effluent, the rate or volume of introduction being selected to attain a desired residence period during which hydrogenating fluid is in contact with the catalyst. A sample of continuously withdrawn effluent or of the hydrogenating vessel fluid contents is conveniently monitored for indication of progress of step b). For example hydrogenating fluid in the sample may be monitored whereby a diminished consumption of hydrogenating fluid in a reactor vessel operated in regeneration mode, or in a regeneration vessel having batchwise introduction of catalyst to be regenerated, or a constant consumption of hydrogenating fluid in a regeneration vessel having continuous introduction of catalyst to be regenerated and of hydrogenating fluid, indicates that hydrogenation is diminished or complete to the extent possible under the prevailing conditions. Analagously hydrocarbonaceous effluent component may be monitored for a decrease in production. Suitable techniques for monitoring effluent composition are known in the art and include gas chromatographic separation techniques in combination with flame ionisation detection techniques.

A suitable flow rate or residence time of hydrogenating fluid may be determined to attain a desired rate of hydrogenation, taking into consideration i.a concentration or partial pressure of available hydrogen in the hydrogenating fluid and nature and concentration of hydrogenating function, temperature to which the deactivated catalyst is subjected in step b), nature of catalyst composition, for example the concentration of zeolite crystals in the catalyst and the extent of zeolite deactivation on completion of each step a).

By the process of the invention it is possible to operate at an acceptable conversion, i.e. obtaining an acceptable alkylate yield for the duration of the time on stream. It would appear that the catalyst alkylation activity is upheld throughout partial deactivation and until full deactivation of all sites has occurred, at which point olefin breakthrough is observed. Hence for the duration of time on stream it would appear that hydrocarbonaceous deposits remain associated with the acid sites of the catalyst and are not detected in the product. In order to maintain a higher production rate averaged over a process cycle it may be advantageous to operate to a minimum production rate corresponding to partial deactivation of catalyst in combination with a high frequency of deposit removal step b). Rate of alkylate production per weight of catalyst averaged over one full reaction cycle may conveniently be calculated as the product of olefin space velocity, time on stream in step a) and yield of alkylate on olefin, as a function of combined time of one full cycle. Yield of alkylate at effective olefin conversion is taken for this purpose to be 200 %.

A preferred operation of the process of the invention is in the preparation of highly branched paraffins containing 5 to 12 carbon atoms, preferably containing 5,6,7,8,9 or 12 carbon atoms, most preferably trimethyl-butane, -pentanes or -hexanes. The process may be applied in the upgrading of a paraffinic feedstock comprising iso-paraffins having from 4 to 8 carbon atoms as desired, suitably of a feedstock comprising isobutane, 2-methylbutane, 2,3-dimethylbutane, 3-methylhexane or 2,4-dimethylhexane, most preferably a paraffinic feedstock comprising iso-paraffins having 4 or 5 carbon atoms. Suitable feedstocks for the process of the invention include iso-paraffin-containing fractions of oil conversion products such as naphtha fractions, and refined iso-paraffin feedstocks such as refined iso-butane.

The olefins containing stream suitably comprises a lower olefin containing hydrocarbon which may optionally contain additional non-olefinic hydrocarbons. Suitably the olefins containing stream comprises ethene, propene, iso- or 1- or 2-(cis or trans) butene or pentene or mixtures thereof optionally diluted for example with propane, iso-butane, n-butane or pentanes. In a further advantage of the present invention, it is found that the process may be operated with such mixed olefin streams and with pentene streams, whereby refinery fractions may be employed directly as feedstocks, despite the more rapid catalyst deactivation with such feeds by virtue of the case of regeneration according to the present invention. The process of the invention may suitably be operated downstream of a fluid catalytic cracking unit, an MTBE etherification unit an olefin isomerisation unit or an olefin dimerisation unit.

Preferably step a) is conducted in such a manner as to control initial contact of the catalyst with olefin feed. Suitably the reactor is first charged with paraffinic feedstock until full saturation. Internal or external circulation may advantageously be operated as described in copending European patent application numbers 92201015 and 92201016 for high dilution of olefin which is continuously and quantitatively charged at a paraffin to olefin ratio greater than 5 to the reactor at an acceptable olefin space velocity. Suitable olefin space velocity is such that conversion is maintained in excess of 90 mol%. The feed mixture may advantageously be charged in known manner for improved dispersion for example via a plurality of inlet ports.

For transition from step a) to step b) in a reactor or bed operated in batchwise process, the supply of olefin is terminated or is switched to a feed line operated in parallel supplying a bed operated in antiphase with respect to the cyclic process. Remaining alkylate entrained in the reactor or bed is removed, for example by terminating supply of paraffinic feedstock and drying the reactor or bed at elevated temperature and/or reduced pressure, or by continuing supply of hydrocarbonaceous feedstock as purge for remaining alkylate in the reactor whereafter the supply of hydrocarbonaceous feedstock is also terminated. The catalyst is then contacted with hydrogenating medium, for example by feeding hydrogenating fluid continuously with optional recycle or for a period to attain a desired partial pressure in the reactor as above described.

For transition from step a) to step b) of a process operated with use of a slurry reactor and continuous catalyst regeneration, catalyst is periodically or continuously withdrawn from the reactor vessel and passed to a separate vessel in which hydrogenating fluid is periodically or continuously introduced and effluent withdrawn accordingly. Removal of entrained effluent from step a) may be effected within the hydrogenation vessel by drying at elevated temperature and/or reduced pressure or by subjecting catalyst prior to hydrogenation with a non-olefinic hydrocarbonaceous purge for removal of entrained alkylation effluent. In a continuous withdrawal process, the purge may be carried out in a separate vessel prior to passing catalyst to the hydrogenation vessel.

For repeat of the batchwise cyclic process, the supply of paraffinic feedstock is recommenced for a period to saturation of the catalyst, whereafter the supply of mixed paraffinic feedstock and olefin is recommenced as above described. For reuse in step a) of catalyst withdrawn from a slurry reactor for operation of step b), saturation with paraffinic feedstock may be carried out in a separate vessel prior to returning catalyst to the reactor.

Where it is desired to operate with internal or external circulation for example as described in copending European patent application numbers 92201015 and 92201016, the extent thereof may be selected according to the desired frequency and extent of removal of hydrocarbonaceous deposit. A high degree of circulation promotes a greater catalyst lifetime or a low frequency of removal of hydrocarbonaceous deposit.

It may be desirable to convert alkylate further to other products. Accordingly the process of the invention may be operated with use of a feed line for introducing alkylate to a further process unit.

Step a) of the process of the invention may conveniently be carried out at a temperature less than 150 °C, for example at 60 to 100 °C, preferably at 70 to 80 °C, for example at 75 °C. Reactor pressure may be between 1 and 40 bar, preferably between 10 and 30 bar. Suitably reactor pressure is just greater than the vapour pressure of the paraffin so as to maintain the reaction in the liquid phase.

Paraffin to olefin ratio is suitably in excess of 5 v/v, preferably in the range of 10 to 50 v/v, more preferably in the range of 12 to 30 v/v. A low paraffin to olefin ratio suitably within the range 5 to 10 v/v favours the formation of higher alkylates, such as isoparaffins containing 12 carbon atoms. Operation at high paraffin to olefin ratios favours the formation of lower alkylates but may incur increased effluent distillation and external circulation costs.

Preferred olefin conversion is at least 95 mol%, more preferably at least 98 mol%, more preferably at least 99 mol%, such as for example 99.5, 99.8 or 99.9 mol%. Most preferably olefin conversion is substantially complete, i.e substantially 100%.

Preferred operating ranges of olefin space velocity include from 0.05 to 10.0 kg/kg.hr, preferably from 0.1 to 5.0 kg/kg.hr most preferably from 0.2 to 1.0 kg/kg.hr.

Step b) of the process of the invention may conveniently be carried out at a temperature and a pressure equal to or greater than that for step a). Whilst use of same temperature and pressure conditions minimises the period for interchange between steps, it is found that the rate of deposit removal increases with increase in temperature and hydrogenating medium pressure, hence at relatively extreme temperature and pressure a relatively long period for interchange between steps may be partially or completely compensated by a relatively short period for deposit removal.

Suitably step b) is carried out at a temperature of greater than 80 °C, preferably greater than 150 °C, more preferably from 200 to 550 °C. Suitably hydrogenating gas is maintained at a partial pressure of 1 to 100 bar, more preferably of 3 to 50 bar, most preferably 5 to 20 bar.

The process may advantageously be operated by monitoring of composition of effluents from steps a) and b) using known means for example as hereinbefore described whereby feed rates and feed lines are adapted in response to multivariable constraint control of a selected operating parameter. In a preferred operation of the process of the invention the effluents are monitored during operation and composition readings fed to an advanced process controller operating on a process model to achieve optimum process efficiency.

The process of the invention is operated with use of a solid acid catalyst comprising zeolite crystals conforming to the structural classification of a zeolite beta, suitably as in Newman, Treacy et al., Proc. R. Soc. London Ser. A 420, 375 or in US 3,308,069, first reported synthesis. Zeolite beta may be prepared using tetraethylammonium hydroxide as a template and as described in Zeolites 8 (1988) 46-53. The zeolite is suitably calcined before use, for example at 550 °C for 2 hours. The silicon-to-aluminium ratio of the calcined material may be selected as appropriate. The zeolite may be ion exchanged for example with ammonium nitrate to give the hydrogen form and optionally calcined to promote acidity.

In a preferred embodiment of the invention the zeolite is further ion exchanged with a hydrogenating metal component as above defined, preferably platinum, palladium or nickel, in an amount of 0.005 - 10 %wt on zeolite, preferably 0.01-1 %wt, more preferably 0.05-0.5 %wt, and subsequently calcined. Preferably the zeolite is subject to reducing conditions prior to use to render the hydrogenating metal component in metal form. Ion exchange with metal components has been found to promote hydrogenation and is of particular advantage with use of relatively extreme temperature in step b).

The zeolite may be employed directly or further combined with a support. For example the zeolite may be present in combination with a refractory oxide that serves as binder material such as silica, alumina, silica-alumina, magnesia, titania, zirconia and mixtures thereof. The weight ratio of refractory oxide and zeolite suitably ranges from 1:99 to 90:10, preferably from 50:50 to 5:95. The binder is suitably combined with zeolite prior to final calcination thereof, the combination optionally extruded and finally calcined for use.

Step b) of the process of the present invention is not limited to application with step a) as herein defined but may be employed in combination with other known processes for catalytic hydrocarbon conversion using a zeolite beta catalyst wherein zeolite deactivation by deposit of hydrocarbonaceous material takes place. Examples of such applications which may be envisaged include isomerisation, hydrocracking, fluid catalytic cracking, alkylation of aromatics, reforming, methanol to olefin conversion and toluene disproportionation.

This process is of particular advantage in view of the broad temperature range of application which be employed. Suitably temperatures may be employed ranging up to 550 °C and greater. The advantage of operation at higher temperatures with corresponding higher rate of hydrogenation and rapid availability of reactivated catalyst, is found to be available since the zeolite beta in the catalyst appears not to be irreversibly deteriorated at high temperatures under the hydrogenating conditions of this process. Accordingly the process is of particular advantage when operated at temperatures of 250 °C and greater.

The process is now illustrated by way of non-limiting example.

### EXAMPLE 1

Catalyst A comprising palladium supported on zeolite beta was prepared using tetraethylammonium hydroxide as a template according to a recipe as described in Zeolites 8 (1988) 46-53. After synthesis, the solids were separated from the liquid, washed with deionized water, dried and calcined at 550 °C for 2 hours. The deionized water, dried and calcined at 550 °C for 2 hours. The silicon-to-aluminium atomic ratio of the calcined material was 14.7. The zeolite was brought into its acidic form by ion-exchange with ammonium nitrate. The catalyst was loaded with 0.1 %wt palladium by ion-exchange with an aqueous solution of palladium tetraamine chloride and finally calcined at 450 °C for 2 hours.

### EXAMPLE 2

60-140 mesh size catalyst A was dried for 2 hours at 200 °C in air then loaded into a stirred autoclave reactor. The reactor was pressure-tested with hydrogen at room temperature, raised to a temperature of 250 °C and maintained at 250 °C for 16 hours in a flow of hydrogen.

The reactor was brought to a temperature for alkylation reaction maintained between 70 and 100 °C and the reactor filled with isobutane to reaction pressure of 24 bar while stirring the reactor contents. On saturation of the reactor with isobutane, an effluent stream was withdrawn and with continuation of stirring of reactor contents, the reactor was fed with a mixed 2-butene and isobutane feedstock (paraffin-to-olefin ratio of 30 v/v) at an olefin space velocity (osv) of 0.2 kg/kg.h. The effluent withdrawn from the reactor was analysed on-line by GLC and separated into an atmospheric liquid and a vapour product. Reaction was continued until butene breakthrough observed in the effluent, the reactor then depressurized and feed switched to pure hydrogen gas. The reactor was pressurized to 24 bar and temperature raised for regeneration to 250 °C for 16 hours while a flow of hydrogen was fed to the reactor. Then the reactor was cooled to the alkylation reaction temperature and the above described operation for the alkylation reaction repeated.

The operation was continued for 9 cycles with no observed loss in catalyst activity over consecutive cycles. During these cycles the olefin space velocity (osv) was varied between 0.1, 0.2 and 0.4 kg/kg.h, the regeneration temperature was varied between 250 and 450 °C and the regeneration time was varied between 4 and 24 hours. The life time of the catalyst during alkylation (defined as the time during which the olefin conversion is above 90%) is given in Table 1.

**TABLE 1**

| Cycle No. | Alkylation OSV life-time | | Regeneration time temperature | |
|---|---|---|---|---|
| | (kg/kg.h) | (hours) | (hours) | (deg. °C) |
| 1 | 0.2 | 25 | 16 | 250 |
| 2 | 0.1 | 58 | 24 | 250 |
| 3 | 0.4 | 10 | 8 | 450 |
| 4 | 0.2 | 26 | 8 | 350 |
| 5 | 0.4 | 9 | 60 | 250 |
| 6 | 0.2 | 27 | 16 | 250 |
| 7 | 0.2 | 25 | 4 | 450 |
| 8 | 0.4 | 10 | 16 | 250 |
| 9 | 0.1 | 57 | | |

Typically alkylate of substantially uniform quality was produced for each cycle throughout the life time of the catalyst. The yield of essentially paraffinic C5+ product was greater than 190% by weight on olefins. Analysis of the product is given in Table 2.

**Table 2**

| Analysis of the alkylate produced in cycle 6 (Table 1) | |
|---|---|
| Alkylate | Yield (%wt) fraction |
| C5/C5+ | 6 |
| C6/C5+ | 5 |
| C7/C5+ | 6 |
| C8/C5+ | 79 |
| C9/C5+ | 4 |

From Example 2 it is clear that the catalyst may be regenerated to full activity without apparent dealumination of the zeolite which would result in diminished activity.

### EXAMPLE 3

Catalyst A was loaded into a fixed bed recycle reactor and subjected to a flow of hydrogen at 450 °C and atmospheric pressure for 2 hours. The reactor temperature was lowered for alkylation reaction to about 85 °C and the reactor filled with isobutane to reaction pressure of 28 bar. On saturation of the reactor with isobutane, an effluent stream was withdrawn and recycled at a ratio of 100 v/v versus feed recycle. A mixture of 2-butene and isobutane feedstock (paraffin-to-olefin ratio of 30 v/v) was fed into the reactor at an olefin space velocity of 0.2 kg/kg.h with recycle of reactor effluent at a ratio of 100. Reaction was continued until butene breakthrough observed in the effluent, the reactor was depressurized and feed switched to pure hydrogen gas. The reactor was pressurized to 28 bar and temperature raised for regeneration to 450 °C for 16 hours maintaining flow of hydrogen feed to the reactor. Then the reactor was cooled to the alkylation reaction temperature, feed switched to isobutane and recycle commenced as above described for repeat of the alkylation reaction.

Operation was continued for 5 cycles. Alkylate was produced for 23 - 27 hours in each cycle. Olefin conversion was greater than 99%, i.e substantially complete.

## Claims

1. Cyclic process for catalytically alkylating a paraffinic feedstock and upgrading the activity of deactivated catalyst comprising the steps a) of supplying feedstock and olefin at a paraffin to olefin ratio greater than 5 v/v to a reactor containing a solid acid catalyst and removing effluent comprising alkylate product and b) of exposing the catalyst to a hydrogenating medium and removing hydrocarbonaceous effluent characterised in that the catalyst comprises zeolite beta crystals.

2. Process according to claim 1, wherein olefin comprises propene iso- or 1- or 2-butenes or pentenes or mixtures thereof.

3. Process according to claim 1 or 2, wherein the hydrogenating medium comprises a hydrogenating fluid.

4. Process according to claim 3, wherein the hydrogenating fluid is a gas maintained in step b) at a partial pressure of 1 to 100, preferably 3 to 50, more preferably 5 to 20 bar.

5. Process according to claim 3, wherein the hydrogenating fluid is in admixture with an inert gaseous or liquid diluent or reactive hydrocarbon in a ratio of 99:1 to 1:99 v/v.

6. Process according to any of claims 3 to 5, wherein the hydrogenating fluid comprise hydrogen.

7. Process according to any of claims 3 to 6, wherein the hydrogenating medium additionally comprises a hydrogenating function which is preferably a hydrogenating metal component.

8. Process according to claim 7, wherein the hydrogenating function comprises a metal component selected from nickel, palladium and platinum.

9. Process according to claim 7 or 8, wherein the hydrogenating function is present in an amount of 0.005 - 10 %wt, preferably 0.01 - 1 %wt, more preferably 0.05 - 0.5 %wt on zeolite beta.

10. Process according to any of claims 1 to 9, wherein step b) is carried out at a temperature greater than 80 °C, preferably greater than 150 °C, for example from 200 to 550 °C.

11. Process according to any of claims 1 to 10, wherein the reactor comprises a fixed bed or slurry phase reactor optionally employing external or internal circulation of liquid reactor contents in step a).

12. Process according to any of claims 1 to 11 carried out in a plurality of reactors or reactor beds operated with feed and effluent lines in parallel whereby step a) and step b) are operated in antiphase in at least two beds.

13. Process according to any of claims 1 to 11 carried out in a slurry reactor wherein catalyst is withdrawn continuously or periodically from the reactor to a separate vessel for operation of step b) and returned to the reactor for operation of step a).

## Patentansprüche

1. Cyclisches Verfahren zum katalytischen Alkylieren eines paraffinischen Einsatzmaterials und zum Aufbessern der Aktivität eines desaktivierten Katalysators, umfassend die Stufen a) Zuführen von Einsatzmaterial und Olefin bei einem Paraffin/Olefin-Verhältnis von über 5 Vol/Vol zu einem einen festen sauren Katalysator enthaltenden Reaktor und Abführen eines Alkylatprodukt enthaltenden Abstroms und b) Einwirkenlassen eines Hydriermediums auf den Katalysator und Abführen eines kohlenwasserstoffhältigen Abstroms, dadurch gekennzeichnet, daß der Katalysator β-Zeolithkristalle umfaßt.

2. Verfahren nach Anspruch 1, worin das Olefin Propen, Iso- oder 1- oder 2-Butene oder Pentene oder Gemische hievon umfaßt.

3. Verfahren nach Anspruch 1 oder 2, worin das Hydriermedium ein Hydrierfluid umfaßt.

4. Verfahren nach Anspruch 3, worin das Hydrierfluid ein in Stufe b) auf einem Partialdruck von 1 bis 100, vorzugsweise 3 bis 50, stärker bevorzugt 5 bis 20 bar gehaltenes Gas ist.

5. Verfahren nach Anspruch 3, worin das Hydrierfluid im Gemisch mit einem inerten gasförmigen oder flüssigen Verdünnungsmittel oder reaktionsfähigen Kohlenwasserstoff in einem Volumsverhältnis von 99 : 1 bis 1 : 99 vorliegt.

6. Verfahren nach einem der Ansprüche 3 bis 5, worin das Hydrierfluid Wasserstoff umfaßt.

7. Verfahren nach einem der Ansprüche 3 bis 6, worin das Hydriermedium zusätzlich eine hydrierende Funktion umfaßt, die vorzugsweise eine hydrierende Metallkomponente ist.

8. Verfahren nach Anspruch 7, worin die hydrierende Funktion eine unter Nickel, Palladium und Platin ausgewählte Metallkomponente ist.

9. Verfahren nach 7 oder 8, worin die hydrierende Funktion in einer Menge von 0,005 - 10 Gew.-%, vorzugsweise 0,01 - 1 Gew.-%,stärker bevorzugt 0,05 - 0,5 Gew.-%, bezogen auf β-Zeolith, vorliegt.

10. Verfahren nach einem der Ansprüche 1 bis 9, worin die Stufe b) bei einer Temperatur von größer als 80°C, vorzugsweise größer als 150°C, beispielsweise von 200 bis 550°C, ausgeführt wird.

11. Verfahren nach einem der Ansprüche 1 bis 10, worin der Reaktor einen Festbettreaktor oder Aufschlämmphasenreaktor umfaßt, gegebenenfalls unter Anwendung eines externen oder internen Kreislaufes von flüssigen Reaktorinhalten in Stufe a).

12. Verfahren nach einem der Ansprüche 1 bis 11, das in einer Mehrzahl von Reaktoren oder Reaktorbetten mit parallel betriebenen Speise- und Abstromleitungen ausgeführt wird, wobei die Stufe a) und die Stufe b) in wenigstens zwei Betten in Antiphase betrieben werden.

13. Verfahrern nach einem der Ansprüche 1 bis 11, das in einem Slurryreaktor ausgeführt wird, worin Katalysator kontinuierlich oder periodisch aus dem Reaktor in ein getrenntes Gefäß zur Ausführung der Stufe b) entnommen und zum Reaktor zur Ausführung von Stufe a) zurückgeführt wird.

## Revendications

1. Procédé cyclique d'alkylation catalytique d'une charge de départ paraffinique et d'amélioration de l'activité du catalyseur désactivé, comprenant les étapes consistant à (a) fournir la charge de départ et l'oléfine en un rapport paraffine à oléfine supérieur à 5 v/v à un réacteur contenant un catalyseur acide solide et prélever l'effluent comprenant l'alkylat produit et b) exposer le catalyseur à un milieu d'hydrogénation et prélever un effluent hydrocarboné, caractérisé en ce que le catalyseur comprend des cristaux de zéolite bêta.

2. Procédé suivant la revendication 1, caractérisé en ce que l'oléfine comprend du propène, des iso- ou 1- ou 2-butènes, ou des pentènes, ou leurs mélanges.

3. Procédé suivant la revendication 1 ou 2, caractérisé en ce que le milieu d'hydrogénation est constitué d'un fluide d'hydrogénation.

4. Procédé suivant la revendication 3, caractérisé en ce que le fluide d'hydrogénation est un gaz maintenu au cours de l'étape b) à une pression partielle de 1 à 100 bars, de préférence, de 3 à 50 bars, plus avantageusement, de 5 à 20 bars.

5. Procédé suivant la revendication 3, caractérisé en ce que le fluide d'hydrogénation est en mélange à un diluant liquide ou gazeux, inerte, ou à un hydrocarbure réactif, dans le rapport de 99:1 à 1:99 v/v.

6. Procédé suivant l'une quelconque des revendications 3 à 5, caractérisé en ce que le fluide d'hydrogénation est constitué d'hydrogène.

7. Procédé suivant l'une quelconque des revendications 3 à 6, caractérisé en ce que le milieu d'hydrogénation comprend, en outre, une fonction d'hydrogénation qui est, de préférence, un composant d'un métal hydrogénant.

8. Procédé suivant la revendication 7, caractérisé en ce que la fonction d'hydrogénation comprend un composant d'un métal choisi parmi le nickel, le palladium et le platine.

9. procédé suivant la revendication 7 ou 8, caractérisé en ce que la fonction d'hydrogénation est présente en une proportion de 0,005 à 10% en poids, de préférence, 0,01 à 1% en poids, plus avantageusement, 0,05 à 0,5% en poids, par rapport à la zéolite bêta.

10. Procédé suivant l'une quelconque des revendications 1 à 9, caractérisé en ce que l'on entreprend l'étape b) à une température supérieure à 80°C, de préférence, supérieure à 150°C, par exemple, de 200 à 550°C.

11. Procédé suivant l'une quelconque des revendications 1 à 10, caractérisé en ce que le réacteur est constitué par un réacteur à lit fixe ou un réacteur à phase en suspension utilisant éventuellement une circulation externe ou interne du contenu du réacteur liquide dans l'étape a).

12. Procédé suivant l'une quelconque des revendications 1 à 11, caractérisé en ce qu'on l'entreprend dans une multiplicité de réacteurs ou de lits de réacteur fonctionnant avec des conduites d'alimentation et d'effluent en parallèle, où on fait fonctionner l'étape a) et l'étape b) en antiphase dans au moins deux lits.

13. Procédé suivant l'une quelconque des revendications 1 à 11, caractérisé en ce qu'on l'entreprend dans un réacteur à suspension, où on prélève le catalyseur en continu ou de manière périodique du réacteur pour l'envoyer dans un récipient séparé destiné à la mise en oeuvre de l'étape b) et on le ramène au réacteur pour la mise en oeuvre de l'étape a).
